# EUROPEAN PATENT APPLICATION

(11) **EP 3 636 781 A1**
(43) Date of publication of application: **15.04.2020**
(21) Application number: 18199748.7
(22) Date of filing: 10.10.2018
(51) Int. Cl.: C12Q 1/70, A61K 31/00

(54) **NOVEL SCREENING ASSAYS FOR IDENTIFYING COMPOUNDS RESERSING HIV-1 LATENCY**

(71) Applicant: Heinrich-Pette-Institut Leibniz-Institut für Experimentelle Virologie, 20251 Hamburg (DE)
(72) Inventor: Altfeld, Marcus, 20251 Hamburg (DE); Martrus Zapater, Glòria, 20251 Hamburg (DE)
(74) Representative: Uexküll & Stolberg

(57) **Abstract**

The present invention relates to a method for identifying compounds which reverse HIV-1 latency in a subject and can therefore be therapeutically used for eliminating a latent viral reservoir in a subject infected with HIV-1. Further, the present invention relates to a method for identifying compounds which stabilize HIV-1 latency in a subject and can therefore be therapeutically used for preventing replication and outgrowth of latent HIV-1. In yet another aspect, the present invention relates to a compound that increases or decreases the expression level of the HIV-1 Vpu gene for use in method of treating an HIV-1 infection in a subject in need thereof. In yet another aspect, the present invention relates to a compound that increases or decreases the binding between the HIV-1 Vpu protein and NF-κB for use in method of treating an HIV-1 infection in a subject in need thereof.

## Description

The present invention relates to a method for identifying compounds which reverse HIV-1 latency in a subject and can therefore be therapeutically used for eliminating a latent viral reservoir in a subject infected with HIV-1. In another aspect, the present invention relates to a method for identifying compounds which stabilize HIV-1 latency in a subject and can therefore be therapeutically used for preventing replication and outgrowth of latent HIV-1. In yet another aspect, the present invention relates to a compound that increases or decreases the expression level of the HIV-1 Vpu gene for use in method of treating an HIV-1 infection in a subject in need thereof. In yet another aspect, the present invention relates to a compound that increases or decreases the binding between the HIV-1 Vpu protein and NF-κB for use in method of treating an HIV-1 infection in a subject in need thereof.

### BACKGROUND OF THE INVENTION

Highly active antiretroviral therapy (HAART) has resulted in a dramatic reduction of HIV-1-related morbidity, mortality, and the number of new HIV-1 infections. Inhibition of viral replication and associated immune pathology enables the host's immune system to recover and prevents the development of AIDS.

However, current HAART regiments alone do not lead to eradication of HIV-1, resulting in side effects and development of drug resistance that becomes more apparent with life-long treatment and quick rebounds of viral replication following interruption of HAART (Chun et al., 2010; Palmer et al., 2008; Wong et al., 1997).

It is well-understood that a major obstacle towards a successful cure of HIV-1 infection is the establishment of a latent viral reservoir within CD4+ T cells and monocytes early upon infection, which are often located within "sanctuaries" that are difficult to reach by immune effector cells (Fukazawa et al., 2015). Novel therapies aimed at purging the HIV-1 reservoir employ a so-called "shock and kill" strategy (Archin et al., 2012; Deeks, 2012; Elliott et al., 2014), where latently infected cells are targeted by latency reversing agents (LRAs) ("shock"), enabling recognition and killing of HIV-1-reactivated cells by effector immune cells in the presence of HAART ("kill") (Bullen et al., 2014; Elliott et al., 2014).

As current LRAs target cellular host proteins controlling cell activation, including epigenetic inactivation of host genes, concerns about their potential long-term side effects and toxicity have arisen. While it would be advantageous for LRAs to target viral proteins under toxicity aspects, the role of viral proteins in HIV-1 latency is not well understood. Only the HIV-1 protein Tat has so far been suggested to regulate proviral latency reversal, as it directs transactivation of the HIV-1 LTR promoter (Donahue et al., 2012; Kamori and Ueno, 2017; Mousseau et al., 2015).

Therefore, the identification of novel and safer targets for reversing HIV-1 latency and reducing viral reservoirs presently represents a high priority for HIV-1 research.

### DESCRIPTION OF THE INVENTION

It has now been found that the HIV-1 protein Vpu is involved in maintaining HIV-1 latency, and that Vpu-mediated inhibition of NF-κB signaling is required for HIV-1 latency. HIV-1 Vpu is a polyfunctional protein which is unique to HIV-1. It does not occur in HIV-2 (Cohen et al., 1988; Strebel et al., 1988). The genomic sequence of HIV-1 Vpu of the strain used herein (89.6) is provided herein as SEQ ID NO:1. The amino acid sequence of the HIV-1 Vpu protein is provided herein as SEQ ID NO:3. HIV-1 Vpu has been described to modulate specific cellular proteins for proteosomal degradation, such as the viral receptor CD4 (Magadan et al., 2010), and the restriction factor tetherin (Kmiec et al., 2016). Vpu also functions as a viral viroporin and serves as an ion channel for monovalent ions (Strebel, 2014). HIV-1 Vpu interacts and down modulates HLA-C surface expression in acute infection and thus, avoid cytotoxic T lymphocyte (CTL) recognition (Apps et al., 2016).

The identification of HIV-1 Vpu as a viral latency factor allows for a targeted screening of compounds that are able to modulate HIV-1 latency and are useful in therapeutic methods that aim at the latent viral reservoir in HIV-1-infected individuals. The present invention hence provides novel methods of screening for compounds which either increase or decrease the expression level of the HIV-1 Vpu gene. Both groups of compounds are of therapeutic interest. While a compound that increases the expression level of the HIV-1 Vpu gene may be used in therapeutic approaches that seek to permanently lock the virus in the infected cells, a compound that decreases the expression level of the HIV-1 Vpu gene would be useable as a "latency reversing agent" (LRA).

Thus, in a first aspect, the invention relates to an *in vitro* method for identifying a compound which reverses HIV-1 latency in a subject, comprising
(a) contacting a candidate compound with a cell that expresses an HIV-1 vpu gene;
(b) detecting whether said candidate compound alters the expression level of said HIV-1 vpu gene;
wherein a decrease of the expression level of the HIV-1 vpu gene indicates that the compound reverses HIV-1 latency.

In a second aspect, the invention provides an *in vitro* method for identifying a compound which maintains HIV-1 latency in a subject, comprising
(a) contacting a candidate compound with a cell that expresses an HIV-1 vpu gene;
(b) detecting whether said candidate compound alters the expression level of said HIV-1 vpu gene;
wherein an increase of the expression level of the HIV-1 vpu gene indicates that the compound maintains HIV-1 latency.

According to the above methods, the extent of expression of the gene encoding the HIV-1 Vpu protein is assessed. The coding sequence of the HIV-1 vpu gene is depicted in SEQ ID NO:1. Preferably, the methods of the invention which involve the detection of expression levels use the mRNA sequence corresponding to the nucleotide sequence depicted in SEQ ID NO:1. It will be appreciated that the nucleotide sequence of the HIV-1 Vpu gene can exhibit a number of sequence deviations resulting, e.g., from naturally occurring polymorphisms. Encompassed by the meaning of the term "vpu gene" are thus also polynucleotides which result in an mRNA molecule exhibiting at least about 80%, 85%, 90%, 95%, 96%, 97%, 98%, 99% or more sequence identity to the sequence of SEQ ID NO:1. Computer programs for determining the degree of identity between nucleotide sequences are available, e.g., in the Wisconsin Sequence Analysis Package, Version 8 (available from Genetics Computer Group, Madison, USA) and include, e.g., the programs BESTFIT, FASTA and GAP, which are based on the algorithm of Smith and Waterman. These programs can be used with the standard parameters, as recommended by the manufacturer.

According to the methods of the invention, a candidate compound is contacted with a cell that expresses an HIV-1 vpu gene. Preferably, the cell is latently infected with HIV-1. More preferably, the cell is a latently infected Jurkat cell line, such as cell line J89.

Gene expression levels can be quantified by different methods which are generally known to the skilled person. The determination of the expression of the HIV-1 Vpu gene can be performed either at the transcriptional level or, alternatively, at the translational level. Suitable methods for monitoring expression of the HIV-1 Vpu gene at the transcriptional level include those which allow quantitative or semi-quantitative detection of mRNA levels, for example, quantitative RT-PCR (e.g., TaqMan™ RT-PCR), real-time RT-PCR, e.g. from Fluidigm, or other methods which are generally described, e.g., in Sambrook et al. (eds.) Molecular Cloning: A Laboratory Manual, 2nd ed., Cold Spring Harbor Laboratory Press, 1989.

The detection of expression levels at the transcriptional level usually requires as a first step the isolation of mRNA from the expressing cell, e.g. a cell infected with HIV-1. Methods for isolating RNA, such as mRNA, are well known in the art and are discussed in detail in the literature (see, for example, Sambrook et al. (1989), Molecular Cloning - A Laboratory Manual, Cold Spring Harbor Laboratory, Cold Spring Harbor, New York and Ausubel et al. (1994), Current Protocols in Molecular Biology, Current Protocols Publishing, New York). Such methods regularly involve the lysis of the Vpu expressing cell. Cell lysis may be performed by use of detergents which are capable of disrupting the cells. For example, buffers containing guanidine thiocyanate and/or SDS may be used for cell lysis. The methods may comprise steps in which the DNA of the cells is enzymatically digested in order to obtain pure RNA without traces of DNA which might interfere with monitoring of expression levels. Inhibitors of enzymes which lead to the degradation of the RNA may also be added to the lysis buffer. Kits for preparing highly purified RNA are available from several manufacturers, such as Qiagen, Ambion, Stratagene, Clontech, Invitrogen, Promega, and others.

The RNA isolated from the cell by use of commercial kits will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex™ matrix can be performed, as mRNAs contain a poly(A) tail at their 3' terminus (see, for example, Ausubel et al. (1994), Current Protocols in Molecular Biology, Current Protocols Publishing, New York). Poly(A)+ mRNA which has bound to the affinity matrix can be eluted using 2 mM EDTA/0.1% SDS.

One commonly used method for detecting expression at the transcriptional level is RT-PCR. In this method, an mRNA template is transferred into cDNA by a reverse transcription (RT) reaction. The reverse transcription of the RNA template is catalyzed by a reverse transcriptase. Reverse transcription can be primed by specific oligonucleotide primers, or, alternatively, by oligo-dT primers. The cDNA is then used as template for a subsequent PCR reaction. In the subsequent PCR step, the cDNA is amplified by use of specific oligonucleotide primers and a polymerase enzyme, e.g., a Taq polymerase. In a preferred aspect, the RT-PCR reaction is performed as a real-time RT-PCR, which enables the detection and the simultaneous quantification of amplified DNA in real-time. Quantification occurs either as an absolute number of copies or as a relative amount normalized by use of additional gene expression products. Suitable real-time RT-PCR systems can be purchased from Fluidigm or Roche.

In a further preferred aspect, a TaqMan RT-PCR is used for determining the expression levels of the Vpu gene. The TaqMan RT-PCR is a fluorophore-based RT-PCR method which detects accumulation of a specific, amplified product during PCR. In TaqMan RT-PCR, RNA is first transferred into cDNA by a reverse transcriptase. In the subsequent PCR reaction, a single-stranded oligonucleotide probe is added which is complementary to a segment of 10-60 nucleotides within the DNA template and located between the two PCR primers. A fluorophore and a quencher dye are covalently attached to the 5' and 3' ends of the probe, respectively. Alternatively, the quencher dye can also be attached to an internal nucleotide, while the fluorophore is attached to the 5' or 3' end of the probe or vice versa. The close proximity between fluorophore and quencher dye attached to the probe inhibits fluorescence emission from the fluorophore when said fluorophore is selectively excited. During DNA synthesis in the PCR, the 5' exonuclease activity of the Taq polymerase cleaves the oligonucleotide probe which is hybridized to the template DNA, thereby spatially separating the fluorophore and the quencher dye. Fluorescence is detected during PCR cycling; it is directly proportional to the fluorophore released and the amount of DNA template present in the PCR. Every fluorophore-quencher pair known in the art can be used in the methods of the present invention. Examples of suitable fluorophores are FAM (6-carboxyfluorescin), TET (tetrachlorofluorescin) or VIC. A suitable quencher dye is TAMRA (tetramethylrhodamine). The construction and labelling of oligonucleotides to be used as probes in a TaqMan approach are described in great detail in the literature. A TaqMan approach RT-PCR reaction can be carried out using, e.g., the ABI PRISM 7700 system (Perkin-Elmer/Applied Biosystems, Foster City, Calif., USA), or the Lightcycler system (Roche Molecular Biochemicals, Manheim, Germany).

A microarray is another commonly used tool in expression profiling. A microarray refers to the orderly arrangement of spatially resolved probes, for example nucleic acid probes, on a substrate. Such arrays can contain at least one, and preferably more than one, oligonucleotide which is complementary to the Vpu gene and, thus, hybridizes to the Vpu sequence or its transcripts. The substrate is preferably a solid substrate having a surface with a plurality of probes attached in different known locations (spots). The spots on a microarray are normally either printed on the microarrays or synthesized by photo-lithography or by ink-jet printing. On a common microarray, there may be several thousand spots, wherein each of the spots may contain a high number of identical probes, such as nucleic acid fragments or oligonucleotides. Such microarrays typically have a density of at least 100 oligonucleotides or fragments per cm². In certain embodiments the arrays can have a density of about at least 500, at least 1000, at least 10,000, at least 10⁵, at least 10⁶, at least 10⁷ oligonucleotides or fragments per cm². The support can be a glass or plastic slide or membrane on the surface of which the probes are attached at fixed locations or spots.

The primers or probes used in the PCR or RT-PCR reactions mentioned herein will be designed to allow the specific hybridization to and the subsequent amplification of the target sequence within the Vpu gene. Based on the present disclosure, the skilled person will be readily able to design oligonucleotide primers and/or probes which can be used for detecting the expression of the Vpu gene. Methods for designing sequence-specific oligonucleotide primers for PCR or RT-PCR are discussed in great detail in the scientific literature, e.g. in Dieffenbach and Dveksler, "PCR Primer", A Laboratory Manual, Cold Spring Harbor Laboratory Press, New York, 2003. The parameters to be considered when designing PCR primers include, for example, the number of nucleotides, the G/C content of the primer, the melting temperature, the presence of complementary nucleotides which may lead to secondary structures, and the like. The oligonucleotides for use in the methods of the present invention preferably have a length of at least 8 nucleotides, more preferably, at least 10, 12, 14, 16, 18, 20, 22, 24, 26, 28, 30, 32, 34, 36, 38, 40, 42, 44, 46, 48 or 50 nucleotides.

When assessing whether a candidate compound decreases or increases Vpu gene expression, the expression level determined in the presence of the candidate compound to be tested will be compared to a reference value. The reference value is preferably obtained from the same type of cell under the same conditions except that the reference cell is not in contact with the candidate compound. As a control, the expression level of other HIV-1 genes, such as the gag gene, or housekeeping genes may be quantified.

Where the determination of the mRNA levels reveals that the Vpu gene expression is significantly decreased or increased in the cell that is in contact with the candidate compound relative to the expression level obtained in the reference cell, this indicates that the candidate compound modulates Vpu expression. A significant increase or decrease of the expression level of the Vpu gene means that the Vpu mRNA amounts found in the cell that is in contact with the candidate compound are at least about 25%, about 50%, about 100%, about 200%, about 300%, about 400%, about 500%, or even about 1000% or 2000% higher or lower than the respective amounts in the reference cell. In other words, the expression level of the Vpu gene can be increased or decreased in the cell that is in contact with the candidate compound by at least 2-fold, at least 5-fold, at least 10-fold, or at least 20-fold relative to the expression level in the reference cell.

The detection of expression levels at the translational level may be performed by quantifying Vpu protein levels. Vpu protein levels may be determined by any suitable method which is able to specifically detect the Vpu protein in the cell. The detection of the protein may be based on a molecule that specifically binds to the Vpu protein, e.g. after cell lysis and separation of the Vpu protein from other proteins that are present in the cell.

Molecules that specifically bind to the Vpu protein include antibodies and antibody fragments with binding activity for the Vpu protein. Such antibodies or fragments thereof may be used for detection of the Vpu protein in a Western blot, quantitative Western blot, enzyme-linked immunosorbent-assay (ELISA), polarization analysis, (quantitative) surface plasmon resonance (SPR), or in immunohistochemical methods, including quantitative electronmicroscopic methods. Other methods which are able to detect specific binding include, for example, Förster/fluorescence resonance energy transfer (FRET). Methods which allow the quantitative detection of the Vpu protein by separating the protein from other components in the biological sample include quantitative mass spectrometric methods, electrophoretic methods, such as two-dimensional gel electrophoresis, and chromatographic methods, such as size-exclusion chromatography or ion-exchange chromatography.

If the protein levels reveal that the vpu gene expression is significantly increased or decreased in the cell that is in contact with the candidate compound relative to the reference cell, this indicates that the candidate compound modulates Vpu expression. A significant increase or decrease of the expression level of the Vpu gene means that the Vpu protein amounts found in the cell that is in contact with the candidate compound are at least about 25%, about 50%, about 100%, about 200%, about 300%, about 400%, about 500%, or even about 1000% or 2000% higher or lower than the respective amounts in the reference cell. The protein amounts in the test subject can be increased or decreased by at least 2-fold, at least 5-fold, at least 10-fold, or at least 20-fold relative to the corresponding amounts in the reference cell.

In yet another aspect, the invention pertains to an *in vitro* method for identifying a compound which reverses HIV-1 latency in a subject, comprising
(a) contacting a candidate compound with an HIV-1 Vpu protein and NF-κB;
(b) detecting whether said candidate compound modulates the binding of the HIV-1 Vpu protein to NF-κB;
wherein reduced binding of the HIV-1 Vpu protein to NF-κB in the presence of the candidate compound indicates that said compound reverses HIV-1 latency.

In yet another aspect, the invention pertains to an *in vitro* method for identifying a compound which maintains HIV-1 latency in a subject, comprising
(a) contacting a candidate compound with an HIV-1 Vpu protein and NF-κB;
(b) detecting whether said candidate compound modulates the binding of the HIV-1 Vpu protein to NF-κB;
wherein increased binding of the HIV-1 Vpu protein to NF-κB in the presence of the candidate compound indicates that said compound maintains HIV-1 latency.

The protein binding assays can be designed in many ways. For example, the step in which a candidate compound is contacted with an HIV-1 Vpu protein and NF-κB can take place within a cell, e.g. a cell that has been transfected with a vector that expresses Vpu. For example, an adapted assay based on the dual luciferase reporter assay described in Sauter et al. (2015) may be used to screen Vpu inhibitors affecting the NF-κB pathway activity. HEK293T cells may be co-transfected with: 1) an expression plasmid encoding for a mutant of IKKβ, aiming at activating NF-κB pathway, 2) a reporter construct (e.g. luciferase) containing NF-κB binding sites, 3) a control plasmid for normalization, and 4) a specific amount of a plasmid encoding for Vpu (from several HIV-1 strains). The amount of the plasmid encoding Vpu may be optimized to inhibit NF-κB activity to 50% using the described method in Sauter et al. (2015). Cells can be treated with the candidate compounds and an untreated control can be used for normalization. NF-κB activity will be measured 48h post-transfection/treatment. Compounds increasing NF-κB activity over the 50% threshold (>50%) would be considered as candidates for inhibiting the NF-κB function of Vpu. On the contrary, compounds decreasing NF-κB activity below 50% threshold (<50%) would be considered as candidates for activating or increasing the functionality of Vpu over NF-κB pathway.

Alternatively, the protein binding assays may be performed with labelled Vpu and NF-κB proteins. Preferably, the protein binding assays are carried out with the Vpu protein of HIV-1 depicted in SEQ ID NO:2. Homologs of the protein shown in SEQ ID NO:2 may also be used. As used herein, a homolog of the sequence of SEQ ID NO:2 refers to a polypeptide molecule having a high degree of sequence identity with the amino acid sequence of SEQ ID NO:2. For example, amino acid identity will be at least about 75%, 80%, 85%, 90%, 91%, 92%, 93%, 94%, 95%, 96%, 97%, 98% or 99% when compared in an optimal alignment, for example, by the program BESTFIT using standard parameters. Fragments of the sequence shown in SEQ ID NO:2 may also be used in the assays. These fragments are polypeptides that differ from the amino acid sequence shown in SEQ ID NO:2 (or from the respective homolog sequence) by the absence of one or more amino acids at the N-terminus and/or the C-terminus of the polypeptide. For example, a fragment of the sequence of SEQ ID NO:2 may differ from the sequence of SEQ ID NO:2 by the lack of about 5, 10, 15, 20, or 25, amino acids at the N-terminus and/or the C-terminus, provided that such fragment retains at least a part of the ability of the original full-length VPU protein depicted in SEQ ID NO:2 to bind to NF-κB.

A significant decrease in the binding of Vpu and NF-κB caused by a candidate compound means that binding of Vpu to NF-κB is preferably at least about 25%, about 30%, about 35%, about 40%, about 45%, about 50%, about 55%, about 60%, about 65%, about 70%, about 75%, about 80%, about 85%, about 90%, about 95%, or at least about 100% lower in the presence of a candidate compound than in the absence of said candidate compound, i.e. relative to a control. Stated differently, binding of Vpu to NF-κB in the presence of a candidate compound is preferably reduced by at least 2-fold, at least 5-fold, at least 10-fold, or at least 20-fold relative to binding in the absence of said candidate compound, i.e. relative to a control.

Likewise, a significant increase in the binding of Vpu and NF-κB caused by a candidate compound means that binding of Vpu to NF-κB is preferably at least about 25%, about 30%, about 35%, about 40%, about 45%, about 50%, about 55%, about 60%, about 65%, about 70%, about 75%, about 80%, about 85%, about 90%, about 95%, or at least about 100% higher in the presence of a candidate compound than in the absence of said candidate compound, i.e. relative to a control. Stated differently, binding of Vpu to NF-κB in the presence of a candidate compound is preferably increased by at least 2-fold, at least 5-fold, at least 10-fold, or at least 20-fold relative to binding in the absence of said candidate compound, i.e. relative to a control.

A number of different assay designs can be used to evaluate the effects of a given candidate compounds on Vpu gene expression or Vpu-NF-κB binding. The screening methods are preferably carried out as high throughput screening techniques which allow for the examination of thousands of different candidate compounds in a short period of time. High throughput screening techniques are described in detail in the prior art. The compounds identified by the screening methods can be validated to confirm their activity on gene expression or protein binding.

The candidate compounds used in the screening methods of the present invention can include all different types of organic or inorganic molecules, including peptides, oligosaccharides or polysaccharides, fatty acids, steroids, and the like. For example, the candidate compounds will be small molecules with less than about 2,500 daltons, less than 2000 daltons, less than 1500 daltons, less than 1000 daltons, or less than 500 daltons. Candidate compounds for use in screening methods can be provided in the form of libraries which comprise a high number of synthetic or natural compounds.

In yet another aspect, the invention relates to a compound that decreases the expression level of the HIV-1 vpu gene for use in method of treating an HIV-1 infection in a subject in need thereof, e.g. by reversing HIV-1 latency. In yet another aspect, the invention relates to a compound that increases the expression level of the HIV-1 vpu gene for use in method of treating an HIV-1 infection in a subject in need thereof, e.g. by maintain HIV-1 latency and inhibit virus reproduction.

A compound that decreases the expression level of the HIV-1 vpu gene may be selected from the group consisting of siRNA, shRNA, ribozymes, and antisense RNA. A skilled person will have no problems to prepare inhibitors of Vpu expression based on the Vpu gene sequence. In a particular preferred embodiment, the compound that decreases the expression level of the HIV-1 Vpu gene is siRNA or shRNA. As used herein, a small interfering RNA (siRNA) is a double-stranded RNA structure that typically consists of 15-50 base pairs, more typically 20-25 base pairs. It has a nucleotide sequence which is identical or partially identical to an expressed gene within the cell. siRNA interferes with the expression of said gene via RNA interference, i.e. by degrading mRNA after transcription, thereby preventing translation. Delivery of siRNA can be achieved by transfection, e.g. via cationic liposomes, polymer nanoparticles, or lipid conjugation, by electroporation or by viral vectors.

A short hairpin RNA (shRNA) is an RNA molecule with a tight hairpin turn that may be used to silence gene expression via RNA interference. These molecules have a relatively low degradation rate. They are normally delivered by plasmids or viral vectors and are capable of DNA integration. shRNAs consist of two complementary RNA sequences of 18-24 base pairs that are linked by a short loop of 4-11 nucleotides. Following transcription, the shRNA is exported to the cytosol of the cell where it is recognized by an endogenous enzyme which processes the shRNA into the siRNA duplexes.

Another approach to block gene expression involves the use of antisense oligonucleotides, e.g. antisense RNA or antisense DNA/RNA hybrids. These molecules share sequence identity to their target sequence which facilitates their binding to mRNA to block protein translation. Antisense RNAs typically comprise 15-50 base pairs, more typically 20-25 base pairs. They can be can be synthesized, e.g., by conventional phosphordiester techniques.

In yet another aspect, the invention relates to a compound that decreases the binding between a HIV-1 Vpu protein and NF-κB for use in method of treating an HIV-1 infection in a subject in need thereof, e.g. by reversing HIV-1 latency. In yet another aspect, the invention relates to a compound that increases the binding between a HIV-1 Vpu protein and NF-κB for use in method of treating an HIV-1 infection in a subject in need thereof, e.g. by maintain HIV-1 latency and inhibit virus reproduction.

A small molecule compound that has been found suitable for reversing latency is 1-(2-(azepan-1-yl)nicotinoyl)guanidine (SM111). This compound has been described in the art for being effective in inhibiting HIV-1 replication. However, it was not known that this compound could also reverse HIV-1 latency. In yet another aspect, the invention therefore pertains to 1-(2-(azepan-1-yl)nicotinoyl)guanidine (SM111) or a pharmaceutically acceptable salt or solvent thereof for use in method of reversing HIV-1 latency. The method of reversing HIV-1 latency preferably comprises the administration of an effective amount of SM111 to a subject infected with HIV-1.

In a preferred embodiment, SM111 or a pharmaceutically acceptable salt or solvent thereof, or a compound that decreases the expression level of the HIV-1 Vpu gene, such as a siRNA, shRNA, ribozyme, or antisense RNA directed to Vpu, are used in combination with an antiretroviral compound that is commonly used for HIV treatment. Preferably, this compound is not SM111.

Suitable antiretroviral compounds are those which are presently discussed in the context of a "shock & kill" treatment approach. Antiretroviral compounds which may be used in combination with SM111 include lamivudine, zidovudine, abacavir, tenofovir disoproxil fumarate, tenofovir alafenamide fumarate, emtricitabine, efavirenz, rilpivirine, elvitegravir, cobicistat, dolutegravir, darunavir, and raltegravir. The antiretroviral compounds can be used either alone in combination with SM111 or the VPU expression inhibitor, but in preferred embodiments, combinations antiretroviral compounds will be used together with SM111 or the VPU expression inhibitor.

Preferred combinations include, for example, lamivudine + zidovudine, abacavir + lamivudine + zidovudine, lopinavir + ritonavir, abacavir + lamivudine, tenofovir disoproxil fumarate + emtricitabine, emtricitabine + tenofovir disoproxil fumarate + efavirenz, emtricitabine + rilpivirine + tenofovir disoproxil fumarate, elvitegravir + cobicistat + emtricitabine + tenofovir disoproxil fumarate, abacavir + dolutegravir + lamivudine, atazanavir + cobicistat, darunavir + cobicistat, lamivudine + raltegravir, elvitegravir + cobicistat + emtricitabine + tenofovir alafenamide fumarate, emtricitabine + tenofovir alafenamide fumarate, dolutegravir + rilpivirine, and bictegravir + emtricitabine + tenofovir alafenamide fumarate.

Therefore, in one aspect of the invention the treatment comprises, apart from the administration of the SM111 compound (or the compound that decreases the expression level of the HIV-1 Vpu gene, such as a siRNA, shRNA, ribozyme, or antisense RNA directed to Vpu), also the administration of one or more antiretroviral compounds before, simultaneously with or after administration of SM111 or the VPU expression inhibitor.

Preferably, SM111 (or the VPU expression inhibitor) and the one or more antiretroviral compounds will be present in a single pharmaceutical composition. For the practice of the present invention, it is however not obligatory that both groups of active ingredients are present in a single pharmaceutical composition. Rather, SM111 (or the VPU expression inhibitor) and the one or more antiretroviral compounds can also be present in separate formulations, which are administered to the HIV-1-infected patient to be treated simultaneously or at different times.

When used in a single pharmaceutical composition, the active ingredients of the combination, i.e. SM111 (or the VPU expression inhibitor) and the one or more antiretroviral compounds can be combined, for example, prior to the administration to a patient to give a single delivery form, provided that none of the components shows an intolerable loss in its activity when mixing it with the one or more other component of the combination. For example, SM111 (or the VPU expression inhibitor) and the one or more antiretroviral compound which are present in a lyophilized mixture can be reconstituted to an infusion solution or injection solution by adding a suitable solvent. Moreover, the pharmaceutical composition can be provided as a single dosage form, for example in the form of a pill for the oral administration.

Alternatively, the one or more antiretroviral compounds and SM111 (or the VPU expression inhibitor) can occur in separate formulations which are administered to the patient simultaneously or at different times. For example, the one or more antiretroviral compounds and SM111 (or the VPU expression inhibitor) can be administered at different days of a treatment cycle. The one or more antiretroviral compounds can, for example, be administered daily within a repeating treatment cycle of one week, while SM111 (or the VPU expression inhibitor), or the salt or solvate thereof, is administered only at a specific day of this cycle. If the one or more antiretroviral compounds and SM111 (or the VPU expression inhibitor) are to be administered at different times, it is advisable to provide the active ingredients in separate packaging units (for example in several ampoules). In this context, the antiretroviral compounds and SM111 (or the VPU expression inhibitor) can be present in the same or in different delivery forms, as described in more detail below.

In one embodiment of the invention, SM111 (or the VPU expression inhibitor) is administered to the patient prior to the administration of the one or more antiretroviral compounds. Preferably, the administration of SM111 (or the VPU expression inhibitor) occurs 1 to 24 hours prior to the administration of the one or more antiretroviral compounds. In a preferred embodiment, the administration of SM111 (or the VPU expression inhibitor) occurs 12 to 16 hours prior to the administration of the one or more antiretroviral compounds. In a further preferred embodiment, the one or more antiretroviral compounds are administered to the patient prior to the administration of SM111 (or the VPU expression inhibitor). Preferably, the administration of the one or more antiretroviral compounds occurs 1 to 24 hours prior to the administration of SM111 (or the VPU expression inhibitor). In a particularly preferred embodiment, the administration of the one or more antiretroviral compounds occurs 12 to 16 hours prior to the administration of SM111 (or the VPU expression inhibitor).

Normally, the compositions of the invention comprise, apart from SM111 (or the VPU expression inhibitor) and optional antiretroviral compound(s), one or more pharmaceutically acceptable carriers which are physiologically compatible with the other ingredients of the compositions. The compositions may also include further excipients, binders, diluents or comparable substances. The compositions can be administered in any suitable delivery form known in the art. For example, the compositions can be formulated for the oral, rectal, nasal or parental, including subcutaneous, intramuscular, intravenous and intradermal, administration. Accordingly, the he compositions of the invention can occur in the form of granules, powders, tablets, capsules, syrup, suppositories, injection solutions, emulsions or suspensions.

Suitable pharmaceutical compositions for the administration to humans will typically comprise 0.01 mg to 80 mg of SM111 (or the VPU expression inhibitor) per kilogram body weight of the patient. Preferably, the amount of SM111 (or the VPU expression inhibitor) is in the range of 1 mg to 20 mg per kilogram body weight of the patient, and even more preferably in the range of 5 mg to 15 mg per kilogram body weight of the patient. 10 mg SM111 (or the VPU expression inhibitor) per kilogram body weight are particularly preferred. If the pharmaceutical composition, apart from SM111 (or the VPU expression inhibitor), also comprises an antiretroviral compound, the amount of said antiretroviral compound in the composition will typically be between 0.01 mg and 150 mg per kilogram body weight of the patient. Preferably, the amount of the antiretroviral compound in the compositions of the invention will be in the range of 1 mg to 50 mg per kilogram body weight of the patient, and even more preferred in the range of 5 mg to 25 mg per kilogram body weight of the patient. 15 to 20 mg of the antiretroviral compound per kilogram body weight are particularly preferred.

### BRIEF DESCRIPTION OF THE FIGURES

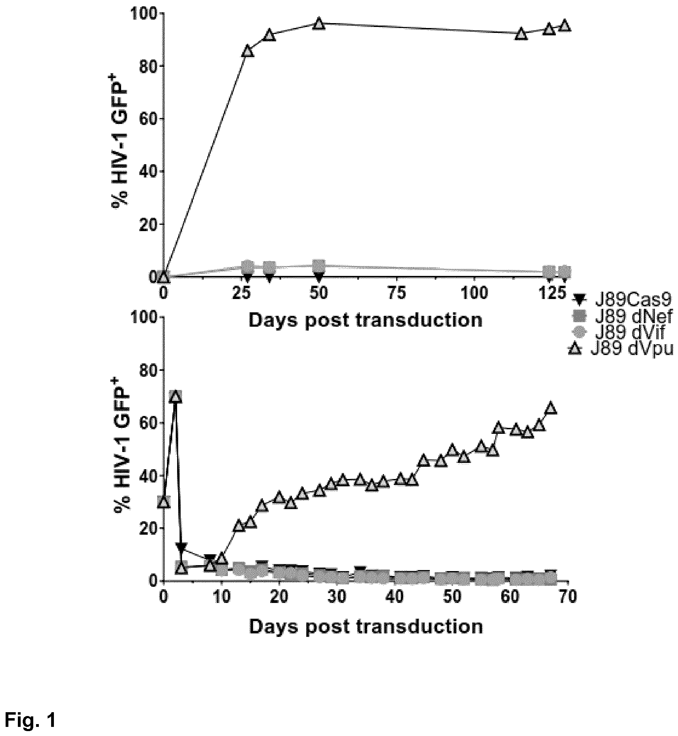

Fig. 1 shows that vpu KO results in latency reactivation in J89 cells. Left panel: J89 were targeted with specific lentiviral constructs containing Cas9 to produce J89Cas9 cells. gRNAs designed against vpu, nef and vif, were transduced in J89Cas9 cells with a lentiviral system (lentiguideRNA) and cells were selected with puromycin and blasticidin. Latency reactivation was traced by quantifying the proportion of HIV-1-GFP+ cells. Right panel: Prior HIV-1 reactivation with TNF-α (10 ng/mL) and subsequent lentiguideRNA transduction led to an initial latency reestablishment in all samples, while only J89Δvpu cells reactivated HIV-1 from latency several days later.

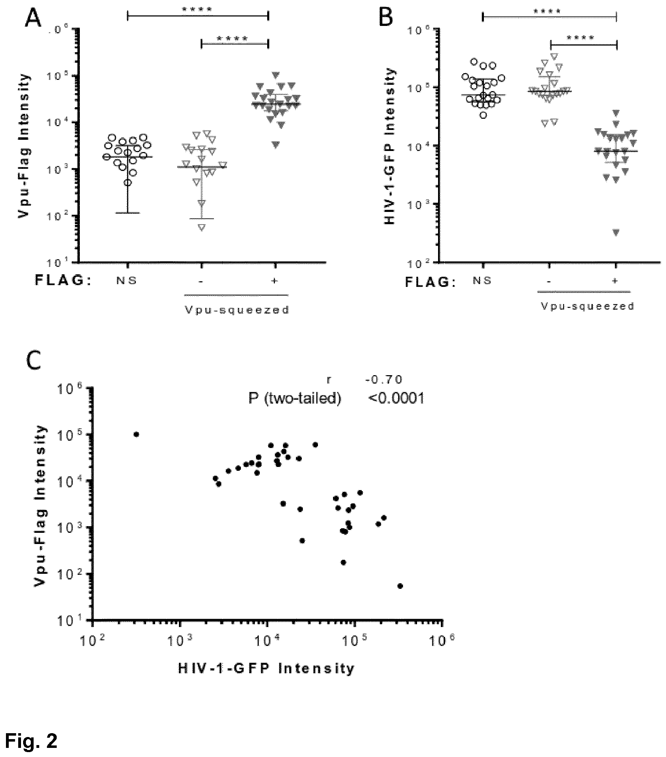

Fig. 2 shows that knocking-in Vpu re-establishes viral latency. (A) Measurement of the Vpu-Flag adjusted intensity (intensity) in NS and Vpu-squeezed cells (positive and negative for the Flag tag), indicating the background level of the staining (n=21 for NS, n=21 for Vpu-squeezed Flag+ and n=20 for Vpu-squeezed Flag-). (B) Measurement of the HIV-1-GFP adjusted intensity (intensity) in NS and Vpu-squeezed cells (positive and negative for the Flag tag), indicating the background level of GFP (n=21 for NS, n=21 for Vpu-squeezed Flag+ and n=20 for Vpu-squeezed Flag-). (C) Correlation of the intensity of the HIV-1 GFP signal with the intensity of the Vpu-Flag signal, on the Vpu-squeezed Flag+ and Vpu-squeezed Flag-samples (n=41). Of note, in A and C, since axes are in logarithmic scale, negative values are excluded from the figure display but not in the calculation. This was the case for 5 samples in the NS condition and 4 samples in the Flag- Vpu-squeezed condition. All results were depicted as medians ± IQR. Statistical tests: Mann-Whitney tests for (A) and (B) (unpaired for NS/SQZ Vpu and paired for -/+ SQZ Vpu, non-parametrical t-test) with ****:p<0.0001 and correlation analyses for (C).

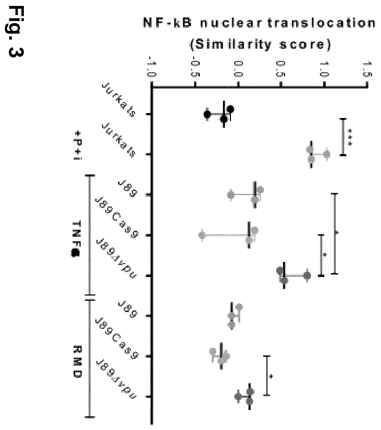

Fig. 3 shows the result from experiments of measuring NF-κB translocation into the nucleus. The mean similarity score of NF-κB translocation into the nucleus was calculated on gated HIV-1-GFP+ cells in TNF-α- and RMD-stimulated samples. Jurkat cells stimulated with PMA and ionomycin (P+i) were used as a positive control (n=3).

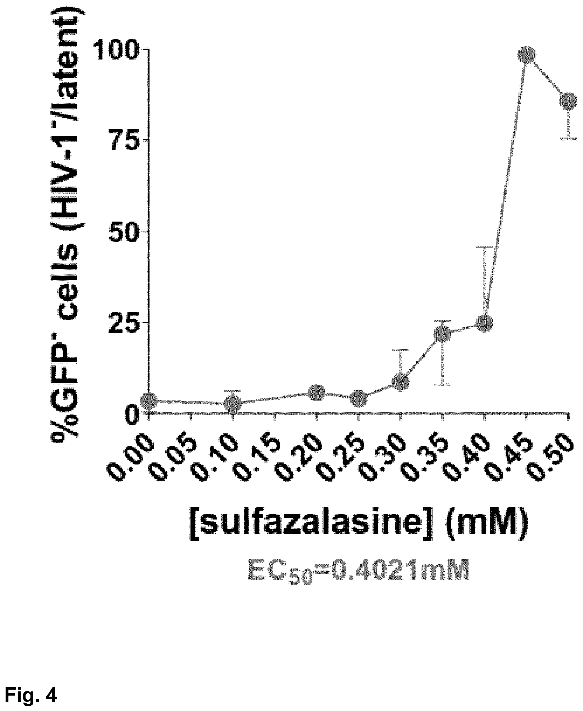

Fig. 4 shows the result from experiments of treating J89Δvpu cells with sulfasalazine. The treatment of J89Δvpu cells with increasing concentrations of sulfasalazine was measured at 96 hrs. Cells shown are gated on viable cells (Zombie NIR- gate) and on negative activated caspase 3 to exclude any apoptotic or dead cell. The proportion of HIV-1-GFP is shown for each specific concentration (n=7).

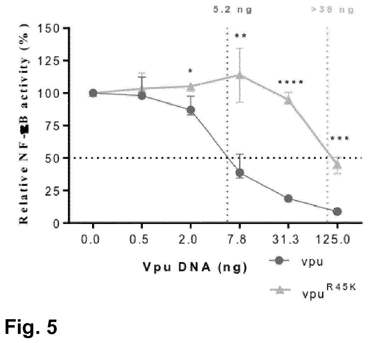

Fig. 5 shows the result from the luciferase reporter assay. It can be seen that the activation of NF-κB at 48 hrs post-HEK293T transfection with a firefly luciferase reporter construct, a Gaussia luciferase construct for normalization, an expression plasmid for a constitutively active mutant of IKKβ and increasing amounts of an expression plasmid encoding for vpu or vpuR45K (n=3).

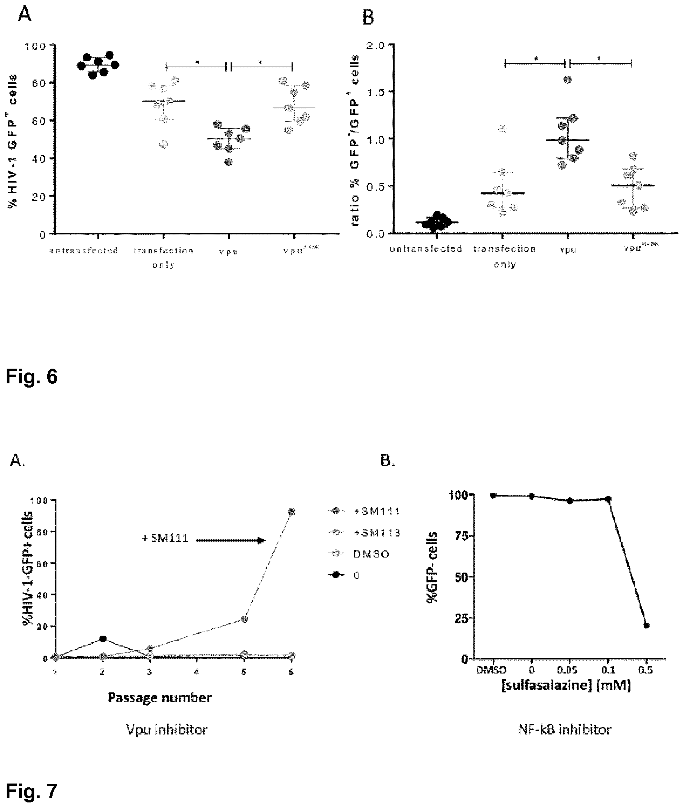

Fig. 6 shows the result from Amaxa transfection and flow cytometry staining. (A) J89Δvpu cells were transfected using Nucleofector™ technology with pCDNA3.1-vpu (vpu) or pCDNA3.1-vpuR45K (vpuR45K) and HIV-1 latency was measured at 96 hrs by flow cytometry on viable cells (Zombie NIR- population) based on the proportion of GFP cells (n=7). Un-transfected and transfected without any plasmid (transfection only) conditions were used in parallel as internal controls (n=7). (B) The ratio between the proportion of GFP- and GFP+ cells in a given sample was calculated and expressed as "ratio % GFP-/GFP+ cells" (n=7). All results were depicted as medians ± IQR. Statistical tests: Mann-Whitney tests (unpaired, non-parametrical t-test) with *:p<0.05, **:p<0.01, ***:p<0.001 and ****:p<0.0001.
Fig. 7 shows the result from testing drug effects on HIV-1 latency. (A) J89 cells were passaged every 3 days with 50 µM of SM111, the control compound SM113, DMSO or left untreated. Latency reversal was quantified as the proportion of HIV-1-GFP+ cells in the cell culture by flow cytometry. (B) NF-κB inhibitor, sulfasalazine, was added at several concentrations to the J89Cas9AVpu cell line and the proportion of GFP- cells (latent) was measured by flow cytometry at 96 hrs. (gating strategy: (A): FSC/SSC → Zombie NIR- → HIV-1-GFP. (B) FSC/SSC → Zombie NIR negative → Caspase 3 active negative → HIV-1-GFP). The caspase 3 active staining was added to exclude early apoptotic cells, as sulfasalazine has shown to activate Caspase 3, and therefore, induce apoptosis.

### EXAMPLES

The following examples are provided in order to illustrate the invention. It should however be understood that the scope of the invention is not limited by the examples. A skilled person will understood that several modifications can be made without deviating from the scope of the invention.

### Example 1: Vpu knockout reactivates HIV-1 from latency

To identify the role of distinct accessory HIV-1 proteins in viral latency and reactivation, a Jurkat T-cell line was used that had been latently infected with the HIV-1 89.6 strain - eGFP tagged (J89 cells) (Martrus et al., 2016), and individual accessory HIV-1 genes were inactivated by a targeted CRISPR/Cas9 knock-out (KO).

Jurkat cells were obtained through the NIH AIDS Reagent Program, Division of AIDS, NIAID, NIH: Jurkat Clone E6-1 from Dr. Arthur Weiss (Weiss et al., 1984) (NIH-ARP Cat# 177-450, RRID:CVCL_0367). J89 cells were a kind gift from Dr. David N. Levy (NYU). J89 is a latent Jurkat cell line containing a single copy of HIV-1 with eGFP between env and nef (Kutsch et al., 2002; Martrus et al., 2016). J89 cells were transduced with lentiCas9 virus, which was a kind gift from Feng Zhang (Addgene plasmid #52962) and produced in HEK293T cells (ATCC Cat# CRL-3216, RRID:CVCL_0063) by spinoculation for 2 hrs at 37°C and 1200 g. The supernatant was discarded and cells were resuspended in fresh media. Selection with blasticidin (5 µg/ml) was initiated 3 days post-transduction and cells were expanded for at least 2 weeks. J89Cas9 cells were then transduced with lentiguideRNA-vpu, lentiguideRNA-nef or lentiguideRNA-vif (Genscript) with a similar procedure described above and puromycin selection (1 µg/ml) was started 3 days post-transduction. Cells were expanded for several weeks in media and HIV-1-GFP expression was monitored via flow cytometry on a BD LRS Fortessa (BD Biosciences).

J89Cas9, J89Δnef, J89Δvif and J89Δvpu cells were cultured for 24 passages, until the percentage of GFP+ J89Δvpu cells was stably above 80%. Single cells were sorted in roundbottom 96-well plates containing 150 µl RPMI+10% FCS (R10) with the respective antibiotics using a FACS Aria Sorter (BD Biosciences). Cells were expanded until reaching a 6 well plate and clones were selected for their GFP expression. None of the J89Δvpu cells initially sorted as GFP+ lost their strong GFP expression. All J89Δvpu and J89Cas9 cells sorted as GFP- maintained their latency over the duration of the experiment. Genomic DNA (gDNA) was extracted using the DNeasy Blood & Tissue Kit (Qiagen). The sequence of vpu was PCR-amplified using the following primers:
vpu-FwdEcoRI - SEQ ID NO:3
   (5'-GAGTCCTAGCGAATTCCAGCGACGAAGACCTCCTCAAG-3'); and
vpu-RevNotI - SEQ ID NO:4
   (5'-CGAGTACTACGCGGCCGCCTGTATCATAGGCTTTAGCATCTGATGCAC-3')
and cloned into pVLX-IRES-puro (Clontech, Takara Bio) vector via NotI/EcoRI ligation. DH5α E. coli were transformed with pVLX-IRES-puro-vpu and bacterial clones were selected and sequenced to identify the variety of vpu mutants (Seqlab). The 89.6 vpu sequence was modified to mutate the vpu-gRNA recognition site and was cloned into pCDNA3.1 (Invitrogen) to generate pCDNA3.1-vpu. Using the QuikChange II XL Site-Directed Mutagenesis Kit (Agilent), the mutation R45K was introduced to produce the pCDNA3.1-vpuR45K construct. All plasmids were sequenced to confirm their mutations (Seqlab).

Results: It was found that a KO of vpu resulted in a complete loss of viral latency, as measured by increased levels of GFP expression, while KO of nef or vif had no impact on HIV-1 latency (Fig. 1A, left panel). To determine whether Vpu was involved in the establishment versus maintenance of latency, the parental J89 (Cas9+) cells were pre-stimulated with TNF-α to induce HIV-1 reactivation, and performed CRISPR/Cas9-mediated KO of the individual HIV-1 accessory genes 24 hrs later. All cell lines, independent of the disrupted HIV-1 accessory gene including vpu, regained HIV-1 latency within 3 days, demonstrating that even the J89Δvpu cells retained their ability to establish latency following HIV-1 reactivation (Fig. 1A, right panel). However, only the J89Δvpu cells subsequently reactivated and exhibited progressively higher rates of HIV-1 replication, strongly implicating vpu in the maintenance of viral latency (Fig. 1A, right panel).

Since targeting of gRNAs on the desired sequence and transduction of lentigRNA are not 100% efficient, J89Δvpu cells were subcloned for subsequent experiments by cell sorting on HIV-1-GFP expression and confirmed KO of vpu by sequencing. An overlay of multiple sequences was detected in HIV-1-GFP+ clones, such as 7E1 (Fig. 1B, top panel), which is likely a consequence of multiple HIV-1 integration events in the host cell genome due to high HIV-1 replication rates. All detected mutations generated a premature stop codon within the targeted vpu sequence, or even a mutation on the methionine in position 1 to an isoleucine, affecting vpu transcription (Fig. 1B, bottom panel). Of note, stably sorted GFP- cells from the overall lentiguideRNA-vpu transduced J89Δvpu cell-pool, such as clone 7D8, contained an intact wild type (89.6) vpu sequence, indicating a direct link of the presence of mutations within vpu and HIV-1 latency reactivation. Taken together, these results suggest that Vpu is involved in latency maintenance in J89 cells.

### Example 2: Reintroducing Vpu re-establishes HIV-1 latency

It was determined whether the inability of J89Δvpu cells to maintain HIV-1 latency could be rescued by re-introducing Vpu protein. The delivery was achieved by the Cell Squeeze® system which, unlike many other techniques, can be used to deliver full proteins directly into cells (Griesbeck et al., 2015). This approach is a membrane-disruption technique using micro-fluidic systems containing parallel constrictions narrower than the cell to disrupt the cell membrane and deliver any protein inside the cell (Stewart et al., 2016).

Briefly, J89Δvpu cells were prepared at 20x10⁶ cells/ml in ice-cold Opti-MEM I reduced medium (Life Technologies) and 100 µl of cells were used per condition. 100 nM of 89.6 Vpu protein synthesized and purified (Acris Antibodies) or 100 nM of a 10 000 MW fluorescent dextran (Texas Red, Life Technologies) were used to treat the cells. Each condition was mixed, and cells were either squeezed on ice at 60 psi through a chip with constriction width of 6 µm and a length of 10 µm or rested on ice (endocytosis, EC, control). A condition without protein was used as an internal negative control. Cells were rested for 3 min, and quenched with fresh R10 for 10 min at 37°C. All samples were centrifuged, washed, resuspended with fresh R10 and cultured at 37°C/5% CO₂. 24 hrs post-squeezing, samples were divided for flow cytometry staining and for immunofluorescence staining. For flow cytometry, cells were stained with α-human CD317 (BST2, Tetherin) (PE, BioLegend Cat# 348406, RRID: AB_10567247) and Zombie NIR (Biolegend) for 20 min at RT and fixed with BD Cytofix/Cytoperm (BD Biosciences) for 20 min at RT. Then, cells were washed twice with BD Perm/Wash and incubated with α-DYKDDDDK Tag Antibody (APC, BioLegend Cat# 637308, RRID:AB_2561497) for 20 min at RT. Cells were washed and acquired at the BD LRS Fortessa (BD Biosciences).

For confocal microscopy and quantification of FLAG-Vpu/GFP, cell-squeezed samples were fixed with BD Cytofix/Cytoperm (BD Biosciences) for 20 min and permeabilized using the BD Perm/Wash kit. Cells were stained with the anti-DYKDDDDK-Tag-APC (BioLegend Cat# 637308, RRID:AB_2561497) for 20 min at RT, washed with PBS+2% FBS and stained with DAPI (5 nM) for 10 min at RT. After washing, cells were cytospined in microscopy slides coated with NaCl 1M and left to dry overnight (Koh, 2013). Samples were imaged at a Nikon Spinning Disk using the x60 objective. Individual cells were distinguished with DAPI and the integrated density (IntDen) for Vpu-Flag and HIV-1-GFP was quantified using Fiji software (ImageJ, RRID:SCR_003070).

Results: HIV-1 89.6 Vpu protein labeled with a C-terminal DDK-FLAG tag (Vpu-Flag) or dextran of similar molecular weight fluorescently labeled with PE-Texas Red (dextran-PE-TxRed; as a control) were squeezed into J89Δvpu cells, and cargo expression levels, as well as tetherin and HIV-1 GFP expression, were measured 24 hrs post-squeezing by flow cytometry. The squeezed samples had detectable levels of dextran-PE-TxRed and Vpu-Flag, respectively. Notably, the proportion of GFP- cells decreased only in cells containing Vpu-Flag, indicating latency re-establishment. The proportion of Vpu-Flag+ cells in the squeezed sample was statistically higher than in the non-squeezed endocytosis control, which controls for non-specific surface membrane labeling with the Vpu-Flag protein. Moreover, Vpu-Flag+ squeezed cells down modulated tetherin compared to Vpu-Flag- squeezed controls, demonstrating the functionality of the introduced Vpu protein. Only the re-introduction of Vpu protein into J89Δvpu cells by cell-squeezing resulted in a consistent re-establishment of HIV-1 latency in a median of 55% of cells (gated within the Vpu-Flag+ squeezed cells), while none of the other controls showed any significant reduction in GFP expression, indicating ongoing HIV-1 gene expression. The proportion of intracellular 89.6 Vpu detected via the Flag-tag significantly and inversely correlated with the proportion of GFP+ cells (r=-0.88, p=0.0003). Taken together, these data demonstrate that the accessory protein Vpu has an important role in HIV-1 latency control.

To investigate the localization of Vpu-Flag, squeezed J89Δvpu cells stained for Vpu-Flag, DAPI and GFP were visualized using fluorescent microscopy. A specific Vpu-Flag signal was observed intracellularly and was only present in GFP- (or low-expressing) squeezed cells. The intensity of Vpu-Flag staining was significantly stronger in Vpu-Flag+ squeezed cells compared to the background levels of Vpu-Flag observed in non-squeezed (NS) control or Vpu-Flag- squeezed cells (Fig. 3A). The intensity of HIV-1-GFP signal was significantly decreased in Vpu-Flag+-squeezed cells (Fig. 3B), and intensity levels of Vpu-Flag significantly inversely correlated with the intensity levels of HIV-1-GFP (r=-0.70, p<0.0001) (Fig. 3C), confirming the results obtained by flow cytometry. Taken together, re-introduction of Vpu protein into J89Δvpu cells resulted in the partial reestablishment of HIV-1 latency.

### Example 3: Vpu controls HIV-1 latency through NF-κB

To study the mechanism underlying Vpu-mediated control of HIV-1 latency, the function of Vpu as a modulator of NF-κB was explored. Therefore, changes in the nuclear translocation of the NF-κB subunit p65 were examined between J89Δvpu and parental cell lines, as p65 translocation is a correlate of NF-κB pathway activation. NF-κB translocation was measured 24 hrs post-stimulation with the HDAC inhibitor RMD (5 nM) or TNF-α (10 ng/mL). As controls for p65 nuclear translocation, unstimulated Jurkat cells and Jurkat cells stimulated with PMA/ionomycin (P+i) were used (Maguire et al., 2011; Maguire et al., 2015). Following staining for nuclei (DAPI) and p65, cells were analyzed with ImageStream technology as described previously (Maguire et al., 2011; Maguire et al., 2015).

For ImageStream X Mark II Imaging Flow cytometer measurement, 3x10⁶ J89, J89Cas9 and J89Δvpu cells were stimulated either with romidepsin (RMD) (5 nM) or TNF-α (10 ng/ml) as described (Martrus et al., 2016). Jurkat cells used as a positive control for p65 translocation in the nucleus were stimulated for 30 min with Phorbol Myristic Acetate (PMA) (50 ng/µl) and Ionomycin (Iono) (3.75 µM) (Maguire et al., 2011). Cells were fixed with 4% PFA for 30 min and washed with permeabilizing solution (2% FBS, 0.1% Triton-X 100, 0.1% Sodium Azide). Then, cells were stained with the Rabbit α-human NF-κB p65 antibody (MyBioSource Cat# MBS440031, RRID:AB_2178869) for 20 min, washed and incubated with AF647-conjugated Affinipure F(ab')2 Fragment Donkey anti-rabbit IgG (H+L) (Jackson ImmunoResearch Labs Cat# 711-606-152, RRID:AB_2340625) for 20 min at RT. Cells were washed and DAPI staining was done just prior acquisition at the ImageStream® X Mark II Imaging Flow cytometer platform (Merck Millipore). At least 20 000 events were recorded per condition and the similarity score (S) was calculated between the signal from the nucleus (DAPI) and the signal from p65 staining using the IDEAS 6.2 software (Maguire et al., 2011).

Results: Representative images of each sample showed higher levels of nuclear-associated p65 signal (DAPI/p65) for PMA/ionomycin-stimulated Jurkat and J89Δvpu samples, indicating nuclear translocation of p65, and a higher GFP intensity in stimulated-J89Δvpu cells compared to stimulated-J89 and -J89Cas9 cells, as observed in flow cytometry. Using the similarity score, it was confirmed that PMA/ionomycin-stimulated Jurkat cells increased the translocation of p65 into the nucleus compared to unstimulated cells (Maguire et al., 2011). Moreover, J89Δvpu cells stimulated with RMD or TNF-α had a higher p65-similarity score in the nucleus compared to both parental cell lines, J89 and J89Cas9 (Fig. 3). These data show that knockout of Vpu increases NF-κB signaling, implicating Vpu-NF-κB interactions as a potential mechanism of latency maintenance.

### Example 4: Sulfasalazine treatment

Based on the finding that NF-κB activation in J89Δvpu cells is associated with the loss of HIV-1 latency, it was next investigated whether inhibition of NF-κB in J89Δvpu resulted in latency re-establishment. To this end, the NF-κB inhibitor sulfasalazine was used (Doering et al., 2004; Liptay et al., 1999; Liptay et al., 2002). As sulfasalazine induces caspase-3 activation at high doses, J89Δvpu cells were treated with increasing concentrations of sulfasalazine and stained for activated caspase-3 to ensure gating on live, non-early apoptotic cells.

Specifically, J89Δvpu cells (3x10⁶ cells) were treated with increasing concentrations of the NF-κB inhibitor sulfasalazine (Sigma Aldrich, ranging from 0.05 mM to 0.5 mM for 96 hrs, and DMSO was used as an internal toxicity control. Cells were stained with Zombie NIR (Biolegend), α-human CD317 (BST2, Tetherin) (PE, BioLegend Cat# 348406, RRID:AB_10567247) and α-caspase 3 activated (AF647, BD Biosciences Cat# 560626, RRID:AB_1727414) using the BD Cytofix/Cytoperm protocol described above. To quantify the toxicity of sulfasalazine, the LD50 was calculated by plotting the proportion of Zombie NIR-/activated Caspase 3- cells from the parent population against the concentration of sulfasalazine. The effective concentration (EC50) was calculated gating on Zombie NIR/activated Caspase3 negative cells (live cells) and plotted against the concentration of sulfasalazine.

Results: It was found that cells regained viral latency in a dose-dependent manner as measured by an increased proportion of GFP- cells (Fig. 4). The calculated EC50 for HIV-1 latency re-establishment was 0.40 mM of sulfasalazine, whereas toxic doses were only reached at higher doses of sulfasalazine (LD50 > 0.50 mM).

### Example 5: NF-κB reporter assay

It was examined next whether the specific effect of Vpu on NF-κB is critical for HIV-1 latency maintenance. It was previously shown that an arginine (R) to lysine (K) mutation at position 45 (R45K) of Vpu abrogates NF-κB inhibition activity (Yamada et al., 2018). Thus, an R45K mutant of HIV-1 89.6 vpu (vpuR45K) was generated, and its ability to modulate NF-κB activity compared to wild-type vpu was assessed using a HEK293T-cell based luciferase reporter assay.

Briefly, the Vpu-mediated modulation of NF-κB activation was monitored using a dual luciferase reporter assay (Sauter et al., 2015). HEK293T cells were co-transfected with a firefly luciferase reporter construct under the control of three NF-κB binding sites (100 ng), a Gaussia luciferase construct under the control of a minimal pTAL promoter for normalization (5 ng), an expression plasmid for a constitutively active mutant of IKKβ (40 ng) to activate NF-κB, and increasing amounts of an expression plasmid for HIV-1 Vpu (0.0 ng, 0.5 ng, 2.0 ng, 7.8 ng, 31.3 ng, 125.0 ng). Cells were transfected in triplicates in 96-well plates, using the calcium phosphate method. 48 hrs post-transfection, firefly and Gaussia luciferase activities were determined using the Luciferase Assay System (Promega) and a homemade assay, respectively. Firefly luciferase signals were normalized to the respective Gaussia luciferase control, and the values obtained in the control without Vpu were set to 100%.

Results: It was found that Vpu inhibits NF-κB activity and that vpuR45K displayed significantly decreased anti-NF-κB activity compared to vpu wild type (EC50 > 38 ng and EC50 = 5.2 ng, respectively) (Fig. 5).

### Example 6: Amaxa transfection and flow cytometry staining

To evaluate the impact of vpuR45K on HIV-1 latency, J89Δvpu cells were transfected with pCDNA3.1-vpu and pCDNA3.1-vpuR45K using an electroporation system (Amaxa Cell Nucleofector). For this purpose, J89Δvpu cells were transfected using the Amaxa® Cell Line Nucleofector® Kit V optimized for Jurkat E6-1 cells (Lonza). In short, 1x10⁶ cells were prepared in 100 µl of Nucleofector® solution and mixed with 2 µg of the corresponding plasmid, pCDNA3.1-vpu and pCDNA3.1-vpuR45K, or left without plasmid. Cells were transferred in the cuvettes and electroporated using the program X-05 for high expression level on a Nucleofector® I device. After 10 min incubation, fresh media was added to the cells and let to rest in the incubator for 96 hrs. Flow cytometry staining as described above with α-human CD317 (BST2, Tetherin) (PE, BioLegend Cat# 348406, RRID:AB_10567247) and Zombie NIR (Biolegend) was performed.

Results: In line with the Vpu protein squeezing results, reintroduction of vpu re-establishes HIV-1 latency in approximately 51% of J89Δvpu cells as compared to the transfection only control (26.5%) (Fig. 6A). Transfection with pCDNA3.1-vpuR45K did not have an impact on re-establishment of HIV-1 latency, as the percentage of HIV-1-GFP+ cells did not differ significantly from the transfected only control (Fig. 6A). The GFP-/GFP+ ratio using pCDNA3.1-vpu was significantly higher than the ratio observed using pDNA3.1-vpuR45K or electroporated-only conditions, reflecting a higher proportion of cells in latency (Fig. 6B). Altogether, our data demonstrate a significant role of Vpu in HIV-1 latency maintenance, relying upon the ability of Vpu to suppress the activation of NF-κB.

### Example 7: SM111 treatment

For SM111 treatment, the HIV-1 latent Jurkat cell line, J89, was treated with SM111 compound, a Vpu-inhibitor, SM113, a negative compound, and DMSO for several consecutive passages. 1x10⁶ cells were plated and treated with 100 µM of all compounds for 4 days. Then, one fourth (25%) of the cells were passaged into new media containing the respective treatment and the rest were used to measure HIV-1 latency reactivation (GFP) and cell viability. An untreated control well was kept as internal negative control.

Results: As can be seen in Figure 7A, only SM111, but none of the other compounds was able to reverse HIV-1 latency.

### LITERATURE

Apps et al. (2016). HIV-1 Vpu Mediates HLA-C Downregulation. Cell Host Microbe 19, 686-695.
Archin et al. (2012). Administration of vorinostat disrupts HIV-1 latency in patients on antiretroviral therapy. Nature 487, 482-485.
Bullen et al. (2014). New ex vivo approaches distinguish effective and ineffective single agents for reversing HIV-1 latency in vivo. Nat Med 20, 425-429.
Chun et al. (2010). Rebound of plasma viremia following cessation of antiretroviral therapy despite profoundly low levels of HIV reservoir: implications for eradication. AIDS 24, 2803-2808.
Cohen et al. (1988). Identification of a protein encoded by the vpu gene of HIV-1. Nature 334, 532-534.
Deeks, S.G. (2012). HIV: Shock and kill. Nature 487, 439-440.
Donahue et al. (2012). The viral protein Tat can inhibit the establishment of HIV-1 latency. J Virol 86, 3253-3263.
Elliott et al. (2014). Activation of HIV transcription with short-course vorinostat in HIV-infected patients on suppressive antiretroviral therapy. PLoS Pathog 10, e1004473.
Fukazawa et al. (2015). B cell follicle sanctuary permits persistent productive simian immunodeficiency virus infection in elite controllers. Nat Med 21, 132-139.
Kamori & Ueno (2017). HIV-1 Tat and Viral Latency: What We Can Learn from Naturally Occurring Sequence Variations. Front Microbiol 8, 80.
Kmiec et al. (2016). Vpu-Mediated Counteraction of Tetherin Is a Major Determinant of HIV-1 Interferon Resistance. MBio 7.
Magadan et al. (2010). Multilayered mechanism of CD4 downregulation by HIV-1 Vpu involving distinct ER retention and ERAD targeting steps. PLoS Pathog 6, e1000869.
Mousseau et al. (2015). The Tat Inhibitor Didehydro-Cortistatin A Prevents HIV-1 Reactivation from Latency. MBio 6, e00465.
Palmer et al. (2008). Low-level viremia persists for at least 7 years in patients on suppressive antiretroviral therapy. Proc Natl Acad Sci U S A 105, 3879-3884.
Sauter et al. (2015). Differential regulation of NF-κB-mediated proviral and antiviral host gene expression by primate lentiviral Nef and Vpu proteins. Cell Rep 10(4):586-99.
Strebel et al. (1988). A novel gene of HIV-1, vpu, and its 16-kilodalton product. Science 241, 1221-1223.
Strebel, K. (2014). HIV-1 Vpu - an ion channel in search of a job. Biochim Biophys Acta 1838, 1074-1081.
Wong et al. (1997). Recovery of replication-competent HIV despite prolonged suppression of plasma viremia. Science 278, 1291-1295.

## Claims

1. *In vitro* method for identifying a compound which reverses HIV-1 latency in a subject, comprising
(a) contacting a candidate compound with a cell that expresses an HIV-1 Vpu gene;
(b) detecting whether said candidate compound alters the expression level of said HIV-1 Vpu gene;
wherein a decrease of the expression level of the HIV-1 Vpu gene indicates that the compound reverses HIV-1 latency.

2. *In vitro* method for identifying a compound which maintains HIV-1 latency in a subject, comprising
(a) contacting a candidate compound with a cell that expresses an HIV-1 Vpu gene;
(b) detecting whether said candidate compound alters the expression level of said HIV-1 Vpu gene;
wherein an increase of the expression level of the HIV-1 Vpu gene indicates that the compound maintains HIV-1 latency.

3. *In vitro* method for identifying a compound which reverses HIV-1 latency in a subject, comprising
(a) contacting a candidate compound with an HIV-1 Vpu protein and NF-κB;
(b) detecting whether said candidate compound modulates the binding of the HIV-1 Vpu protein to NF-κB;
wherein reduced binding of the HIV-1 Vpu protein to NF-κB in the presence of the candidate compound indicates that said compound reverses HIV-1 latency.

4. *In vitro* method for identifying a compound which maintains HIV-1 latency in a subject, comprising
(a) contacting a candidate compound with an HIV-1 Vpu protein and NF-κB;
(b) detecting whether said candidate compound modulates the binding of the HIV-1 Vpu protein to NF-κB;
wherein increased binding of the HIV-1 Vpu protein to NF-κB in the presence of the candidate compound indicates that said compound maintains HIV-1 latency.

5. *In vitro* method according to any of claims 1-4, wherein the cell is a human cell, preferably a T cell.

6. *In vitro* method according to any of claims 1-2, wherein the HIV-1 Vpu gene has the sequence shown in SEQ ID NO:1 or a sequence having at least 80% identity thereto.

7. *In vitro* method according to any of claims 3-4, wherein the HIV-1 Vpu protein has the sequence shown in SEQ ID NO:2 or a sequence having at least 80% identity thereto.

8. *In vitro* method according to any of claims 1-7, wherein the expression level of the Vpu gene is increased at least 2-fold, preferably at least 5-fold, relative to a reference cell.

9. *In vitro* method according to any of claims 1-7, wherein the expression level of the Vpu gene is decreased at least 2-fold, preferably at least 5-fold, relative to a reference cell.

10. Compound that increases or decreases the expression level of the HIV-1 Vpu gene for use in method of treating an HIV-1 infection in a subject in need thereof.

11. Compound for use in a method of claim 10, wherein said compound decreases the expression level of the HIV-1 Vpu gene and is selected from the group consisting of siRNA, shRNA, dsRNA, RNA polymerase III-transcribed DNA, ribozymes, and antisense nucleic acids.

12. Compound for use in a method of claim 11, wherein said compound is siRNA.

13. Compound that increases or decreases the binding between a HIV-1 Vpu protein and NF-κB for use in method of treating an HIV-1 infection in a subject in need thereof.

14. 1-(2-(azepan-1-yl)nicotinoyl)guanidine (SM111) or a pharmaceutically acceptable salt or solvent thereof for use in method of reversing HIV-1 latency.

15. 1-(2-(azepan-1-yl)nicotinoyl)guanidine (SM111) or a pharmaceutically acceptable salt or solvent thereof for use according to claim 11, wherein said method further comprises the administration of an antiretroviral compound.


SEQUENCE LISTING

<110>  Heinrich-Pette-Institut Leibniz-Institut für Experimentelle
       Virologie

<120>  NOVEL SCREENING ASSAYS

<130>  P 106041

<160>  4

<170>  PatentIn version 3.5

<210>  1
<211>  243
<212>  DNA
<213>  Human immunodeficiency virus type 1

<400>  1
atgctttctt tacaaatatt agcaatagta gcattagtag tagcagcaat aatagcaata      60

gttgtgtggt ctatagtatt catagaatat aggaaaatat taagacaaag aaaaatagac     120

agattaattg atagaataag agaaagagaa gaagacagtg gcaatgagag tgaaggagat     180

caggaagaat tggcagcact tgagaggggg catcttgctc cttgggatgt tgatgatctg     240

tag                                                                   243


<210>  2
<211>  80
<212>  PRT
<213>  Human immunodeficiency virus type 1

<400>  2

Met Leu Ser Leu Gln Ile Leu Ala Ile Val Ala Leu Val Val Ala Ala
1               5                   10                  15


Ile Ile Ala Ile Val Val Trp Ser Ile Val Phe Ile Glu Tyr Arg Lys
            20                  25                  30


Ile Leu Arg Gln Arg Lys Ile Asp Arg Leu Ile Asp Arg Ile Arg Glu
        35                  40                  45


Arg Glu Glu Asp Ser Gly Asn Glu Ser Glu Gly Asp Gln Glu Glu Leu
    50                  55                  60


Ala Ala Leu Glu Arg Gly His Leu Ala Pro Trp Asp Val Asp Asp Leu
65                  70                  75                  80


<210>  3
<211>  38
<212>  DNA
<213>  Artificial Sequence

<220>

<223>  oligonucleotide primer

<400>  3
gagtcctagc gaattccagc gacgaagacc tcctcaag                              38


<210>  4
<211>  48
<212>  DNA
<213>  Artificial Sequence

<220>
<223>  oligonucleotide primer

<400>  4
cgagtactac gcggccgcct gtatcatagg ctttagcatc tgatgcac                   48